# FASCICULE DE BREVET EUROPEEN

(11) **EP 4 178 647 B1**
(45) Date de publication et mention de la délivrance du brevet: **04.09.2024**
(21) Numéro de dépôt: 21748925.1
(22) Date de dépôt: 05.07.2021
(51) Int. Cl.: B05B 1/34

(54) **TÊTE DE PULVÉRISATION ET DISPOSITIF DE DISTRIBUTION DE PRODUIT FLUIDE COMPORTANT UNE TELLE TÊTE**
SPRÜHKOPF UND VORRICHTUNG ZUR AUSGABE EINES FLÜSSIGEN PRODUKTS MIT SOLCH EINEM KOPF
SPRAY HEAD AND DEVICE FOR DISPENSING A FLUID PRODUCT COMPRISING SUCH A HEAD

(30) Priorité: 07.07.2020 FR 2007187
(43) Date de publication de la demande: 17.05.2023
(73) Titulaire: APTAR France SAS, 27110 Le Neubourg (FR)
(72) Inventeur: SAVALLE, Matthieu, 76350 OISSEL (FR)
(74) Mandataire: CAPRI
(86) Numéro de dépôt international: PCT/FR2021/051226
(87) Numéro de publication internationale: WO 2022/008824

(56) Documents cités:
- WO-A1-2008/015409
- WO-A1-89/05195
- WO-A1-99/13930
- CN-A- 108 405 208
- US-A1- 2015 165 136

## Description

La présente invention concerne une tête de pulvérisation, son procédé de fabrication, et un dispositif de distribution de produit fluide comportant une telle tête.

Plus particulièrement, la présente invention concerne une tête de pulvérisation réalisée d'une seule pièce monobloc.

Généralement, pour réaliser un profil de pulvérisation en amont de l'orifice de pulvérisation, un insert est inséré dans la tête de pulvérisation.

Ce type de dispositif peut présenter certains inconvénients. Ainsi, le procédé de fabrication requiert la fabrication d'au moins deux pièces, et une étape d'assemblage est ensuite nécessaire. De plus, de par les tolérances de fabrication des pièces à assembler (la tête de pulvérisation et l'insert), le profil de pulvérisation n'est pas toujours strictement identique, et les performances du spray ne sont pas toujours reproductibles.

Les documents WO2008015409 et WO8905195 décrivent des dispositifs de l'état de la technique.

La présente invention a pour but de fournir une tête de pulvérisation pour dispositif de distribution de produit fluide qui ne reproduit pas les inconvénients susmentionnés.

Plus particulièrement, la présente invention a pour but de fournir une tête de pulvérisation avec un profil de pulvérisation qui soit réalisée d'une seule pièce monobloc.

La présente invention a également pour but de fournir une telle tête de pulvérisation qui soit réalisée en une seule étape de fabrication.

La présente invention a également pour but de fournir une telle tête de pulvérisation qui soit simple et peu coûteuse à fabriquer et à assembler.

La présente invention a donc pour objet une tête de pulvérisation pour dispositif de distribution de produit fluide, comportant un corps pourvu d'un orifice de pulvérisation, un canal central étant relié audit orifice de pulvérisation via un profil de pulvérisation comportant une chambre tourbillonnaire disposée directement en amont dudit orifice de pulvérisation, ledit profil de pulvérisation comportant au moins deux canaux courbes s'étendant à partir dudit canal central vers ladite chambre tourbillonnaire, chaque canal courbe se connectant de manière non radiale à ladite chambre tourbillonnaire, pour former un spray en sortie dudit orifice de pulvérisation, chaque canal courbe étant de forme convexe, en s'écartant latéralement vers l'extérieur et axialement vers le haut à partir dudit canal central, puis à partir d'une zone d'inflexion, en revenant latéralement vers l'intérieur et axialement vers le haut en direction de ladite chambre tourbillonnaire.

Avantageusement, ladite tête de pulvérisation est du type nasal, ledit canal central s'étendant axialement et ledit orifice de pulvérisation étant orienté axialement.

Avantageusement, lesdits au moins deux canaux courbes débouchent dans ladite chambre tourbillonnaire en étant régulièrement répartis sur la périphérie de ladite chambre tourbillonnaire.

Avantageusement, chaque canal courbe est de forme arrondie, sans aucun angle vif.

Avantageusement, la section transversale de chaque canal courbe se réduit progressivement entre ledit canal central et ladite chambre tourbillonnaire.

Avantageusement, lesdits au moins deux canaux courbes sont de formes, dimensions et courbures sensiblement identiques entre ledit canal central et ladite chambre tourbillonnaire.

En variante, lesdits au moins deux canaux courbes sont de formes, dimensions et/ou courbures différentes entre ledit canal central et ladite chambre tourbillonnaire.

Avantageusement, ledit profil de pulvérisation comporte deux canaux courbes.

Avantageusement, ledit profil de pulvérisation comporte trois canaux courbes.

La présente invention a aussi pour objet un dispositif de distribution de produit fluide, comportant une partie d'actionnement, un réservoir contenant un produit fluide, une tête de pulvérisation pourvue d'un orifice de pulvérisation, et des moyens de distribution pour distribuer au moins une partie dudit produit fluide à travers ledit orifice de pulvérisation lors d'un actionnement, ladite tête de pulvérisation étant réalisée tel que décrit ci-dessus.

Avantageusement, ledit réservoir contient une seule dose de produit fluide distribuée en un seul actionnement du dispositif.

En variante, ledit réservoir contient au moins deux doses de produit fluide distribuées en plusieurs actionnements successifs du dispositif.

Avantageusement, ladite partie d'actionnement comporte deux éléments d'appui latéraux montés pivotants sur ladite tête de pulvérisation, chaque élément d'appui latéral comportant une partie de came respective comportant chacune une surface de came respective coopérant avec ledit réservoir pour le déplacer axialement vers le haut lors de l'actionnement du dispositif.

La présente invention a également pour objet un procédé de fabrication d'une tête de pulvérisation telle que décrite ci-dessus, ledit procédé comprenant de réaliser ladite tête de pulvérisation par impression additive.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique de côté d'un dispositif de distribution nasale de produit fluide selon un mode de réalisation avantageux,
La figure 2 est une vue schématique de détail en perspective d'une tête de pulvérisation selon un premier mode de réalisation avantageux,
La figure 3 est une vue similaire à celle de la figure 2,
La figure 4 est une vue schématique de dessus montrant une section à travers la tête de pulvérisation des figures 2 et 3,
La figure 5 est une vue similaire à celle de la figure 3, montrant un second mode de réalisation avantageux,
La figure 6 est une vue similaire à celle de la figure 4 montrant une section à travers la tête de pulvérisation de la figure 5,
La figure 7 est une vue schématique en perspective et en transparence d'une tête de pulvérisation selon un troisième mode de réalisation avantageux,
La figure 8 est une vue schématique de côté de la tête de la figure 7,
La figure 9 est une vue schématique de dessus de la tête de la figure 7,
La figure 10 est une vue schématique en perspective et en transparence de dessus de la tête de la figure 7, et
La figure 11 est une vue schématique partielle en perspective et en transparence de la tête de la figure 7.

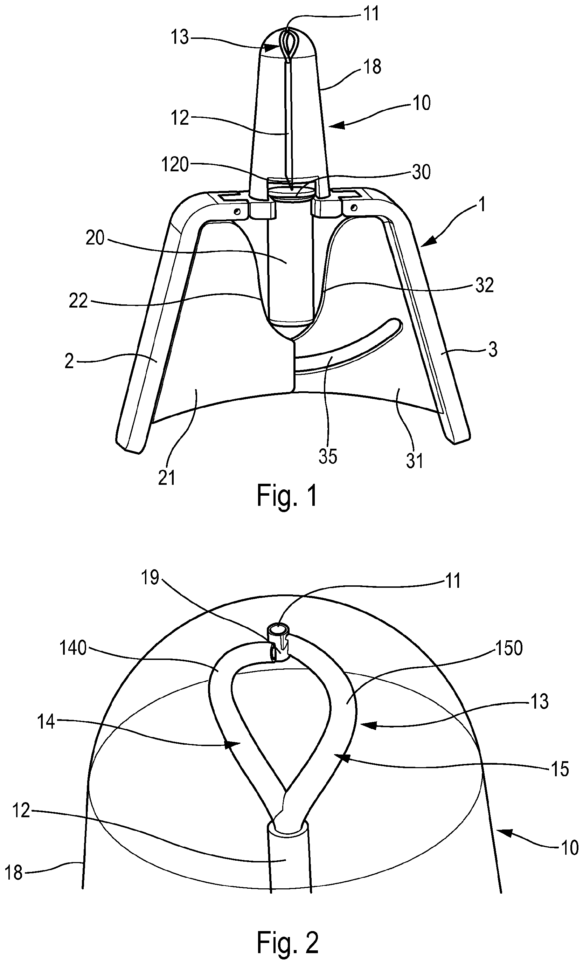

Les termes "proximal" et "distal" se réfèrent à l'orifice de pulvérisation. Les termes "axial" et "radial" se réfèrent à l'axe central longitudinal du dispositif. Les termes "haut", "bas", "supérieur" et "inférieur" se réfèrent à la position droite du dispositif représentée sur la figure 1.

En référence à la figure 1, il est représenté un exemple d'un dispositif de distribution nasale de produit fluide auquel la présente invention s'applique. Dans cet exemple, il s'agit d'un dispositif unidose, dans lequel la totalité de la dose de produit fluide est distribuée en un seul actionnement. Il est à noter que la présente invention pourrait aussi s'adapter à d'autres types de dispositifs, tels que par exemple des dispositifs du type bidose ou multidose, contenant deux ou plusieurs doses de produit fluide à distribuer en deux ou plusieurs actionnements successifs du dispositif.

Dans l'exemple de la figure 1, le dispositif comporte une partie d'actionnement 1, une tête de pulvérisation 10 comportant un corps 18 pourvue d'un orifice de pulvérisation 11, un réservoir 20 contenant le produit fluide à distribuer et des moyens de distribution 30 pour distribuer une dose de produit fluide lors d'un actionnement du dispositif.

La tête de pulvérisation 10, ici du type nasal, comporte un canal central 12 relié audit orifice de pulvérisation 11 via un profil de pulvérisation 13. Cet orifice de pulvérisation 11 sert à distribuer une dose de produit fluide hors de ladite tête de pulvérisation 10 lors de l'actionnement du dispositif par un utilisateur. Une canule 120 est disposée à l'extrémité distale du canal central 12.

Le réservoir 20 est relié à la tête de pulvérisation 10 d'une manière quelconque appropriée. Dans l'exemple représenté sur la figure 1, le réservoir 20 contient une dose unique de produit fluide distribuée en un seul actionnement du dispositif. Typiquement, comme visible sur les figures, le réservoir 20 peut être formé par un corps creux et borgne, comportant une seule ouverture proximale.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon et du côté distal par un second bouchon, le volume défini entre lesdits deux bouchons contenant le produit fluide à distribuer.

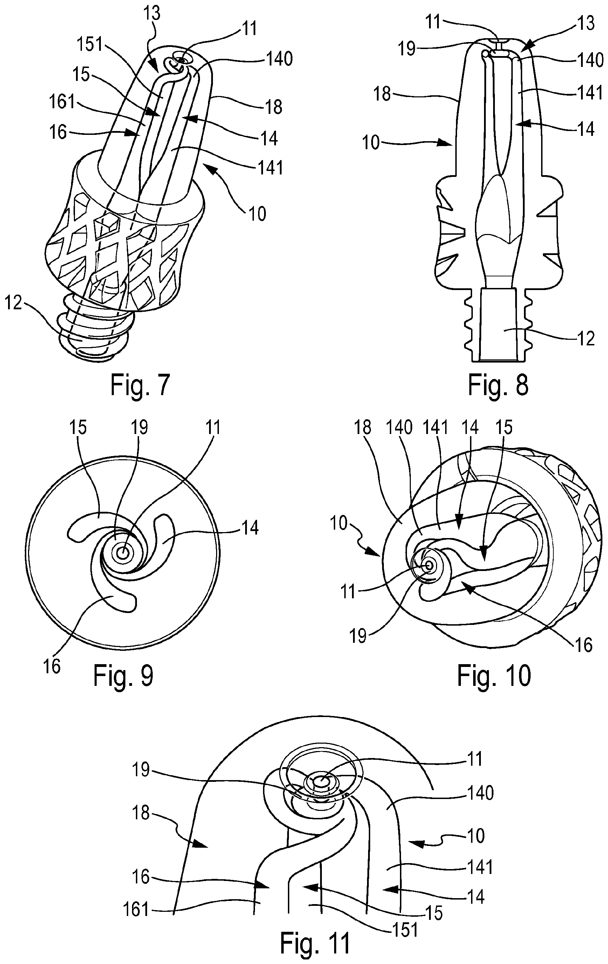

Dans une autre variante, non représentée, le réservoir 20 pourrait être une seringue, sur lequel la tête de pulvérisation se fixe par tout moyen approprié, par exemple une fixation rotative standard du type Luer. Dans l'exemple des figures 7 à 11, la tête de pulvérisation 10 comporte un filetage adapté à se fixer dans une fixation du type Luer d'une seringue.

Dans l'exemple représenté sur la figure 1, qui montre un unidose, les moyens de distribution 30 comportent un bouchon-piston. Ce bouchon-piston 30 obture le réservoir de manière étanche avant actionnement, et est adapté à être percé par la canule 120 en début d'actionnement pour relier le réservoir 20 à l'orifice de pulvérisation 11. La poursuite de l'actionnement déplace ledit bouchon-piston dans le réservoir 20, provoquant ainsi l'expulsion du produit fluide à travers la canule 120 vers l'orifice de pulvérisation 11. Dans un dispositif multidose, les moyens de distribution pourrait comporter une pompe ou une valve doseuse.

La partie d'actionnement 1 comporte dans cet exemple deux éléments d'appui latéraux 2, 3 pour l'appui des doigts de l'utilisateur lors de l'actionnement. Ces deux éléments d'appui latéraux 2, 3 sont montés pivotants sur la tête de pulvérisation 10. Chaque élément d'appui latéral 2,3 comporte une partie de came respective 21, 31, lesdites parties de came 21, 31 étant connectées l'une à l'autre pour permettre un pivotement des éléments d'appui latéraux, par exemple via un ergot (non représenté) de la partie de came 21 coulissant dans une rainure 35 de la partie de came 31. Chaque partie de came 21, 31 comporte une surface de came respective 22, 32 coopérant avec le réservoir 20, notamment avec son bord distal radialement externe, comme visible sur la figure 1. Lors de l'actionnement, l'utilisateur comprime les éléments d'appui latéraux 2, 3 l'un vers l'autre, ce qui provoque leur pivotement, les surfaces de came 22, 32 poussant le réservoir 20 axialement vers le haut pour réaliser la distribution d'une dose de produit fluide.

L'exemple représenté montre donc un actionnement latéral du dispositif, mais il est entendu que la présente invention pourrait aussi s'appliquer à un actionnement classique du type axial.

Selon l'invention, la tête de pulvérisation 10 est réalisée en une seule pièce monobloc, par impression additive.

Ceci permet de réaliser un profil de pulvérisation 13 de forme complexe, difficile voire impossible à réaliser par moulage et/ou assemblage.

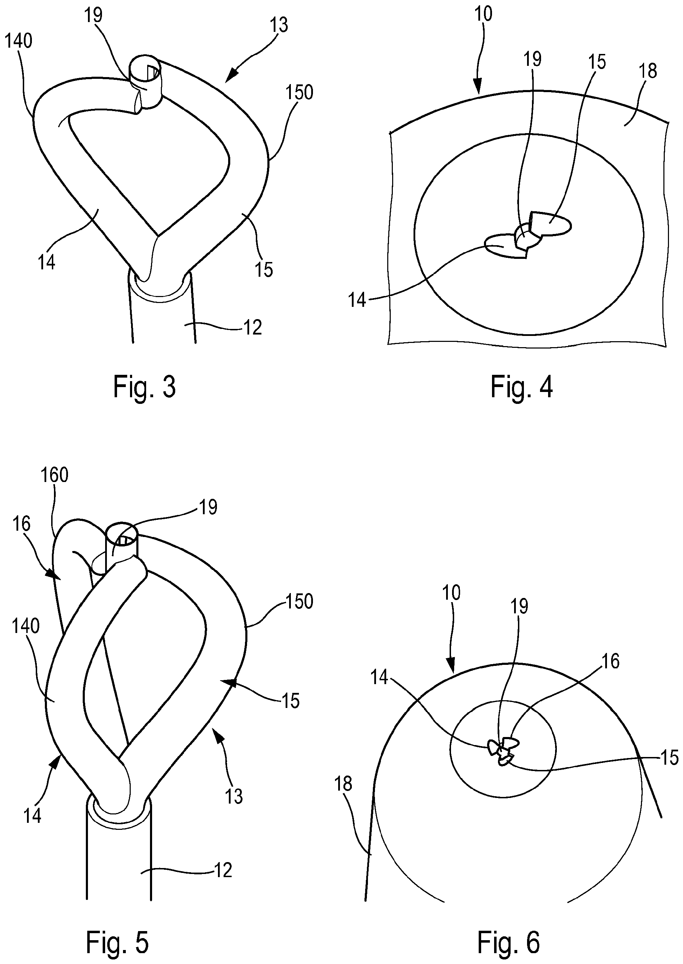

Selon l'invention, le profil de pulvérisation 13 comporte au moins deux canaux courbes 14, 15, 16, s'étendant à partie du canal central 12 vers une chambre tourbillonnaire 19 disposée directement en amont de l'orifice de pulvérisation 11. Les figures 2 à 4 montrent un premier mode de réalisation avec deux canaux courbes 14, 15, les figures 5 et 6 montrent un second mode de réalisation avec trois canaux courbes 14, 15, 16, et les figures 7 à 11 montrent un troisième mode de réalisation avec trois canaux courbes 14, 15, 16. On pourrait envisager un profil de pulvérisation 13 avec plus de trois canaux courbes, par exemple quatre.

Dans l'exemple représenté, qui montre une tête de pulvérisation nasale, le canal central 12 est axial, de même que l'orifice de pulvérisation 11.

Il est à noter que la présente invention pourrait aussi s'appliquer à un dispositif comportant une tête de distribution buccale, avec un orifice de pulvérisation orienté radialement.

Chaque canal courbe 14, 15, 16 se connecte de manière non radiale à la chambre tourbillonnaire 19, générant ainsi un tourbillonnement des flux sortant desdits canaux courbes dans ladite chambre tourbillonnaire 19, formant ainsi un spray en sortie de l'orifice de pulvérisation 11.

De préférence, les au moins deux canaux courbes 14, 15, 16 débouchent dans la chambre tourbillonnaire 19 en étant régulièrement répartis sur la périphérie de celle-ci. Ainsi, dans l'exemple des figures 2 à 4, les deux canaux courbes 14, 15 débouchent de manière diamétralement opposée dans la chambre tourbillonnaire 19, et dans les exemples des figures 5 et 6 et des figures 7 à 11, les trois canaux courbes 14, 15, 16 débouchent dans la chambre tourbillonnaire 19 en étant espacés de 120° les uns des autres. En variante, on pourrait toutefois envisager de faire déboucher les canaux courbes dans la chambre tourbillonnaire en étant répartis d'une manière non régulière, notamment si les dimensions des différents canaux courbes sont différentes.

Comme visible notamment sur les figures 2, 3, 5, 7, 8, 10 et 11 chaque canal courbe 14, 15, 16 est de forme convexe, en s'écartant latéralement vers l'extérieur et axialement vers le haut à partir du canal central 12, puis à partir d'une zone d'inflexion 140, 150, 160, en revenant latéralement vers l'intérieur en direction de la chambre tourbillonnaire 19. Lesdites zones d'inflexion 140, 150, 160 forment avantageusement les parties radialement les plus externes desdits canaux courbes 14, 15, 16.

Dans l'exemple des figures 7 à 11, chaque zone d'inflexion 140, 150, 160 est formée dans une partie axiale supérieure d'une partie axiale de canal 141, 151, 161 qui s'étend sensiblement axialement sur une grande partie de la hauteur de la tête 10. Ainsi, comme visible notamment sur les figures 7, 8 et 10, les trois canaux courbes 14, 15, 16 s'écartent latéralement depuis le canal central 12, puis s'étendent parallèlement les uns aux autres sur leur partie axiale de canal 141, 151, 161 jusqu'à proximité de la chambre tourbillonnaire 19, pour enfin revenir latéralement de manière non radiale vers l'intérieur.

Avantageusement, chaque canal courbe 14, 15, 16 est de forme arrondie, sans aucun angle vif. Ceci permet notamment de limiter les pertes de charges, de sorte que les flux issus des canaux courbes pénètrent dans la chambre tourbillonnaire 19 avec une énergie maximale, générant ainsi un spray d'excellente qualité en sortie de l'orifice de pulvérisation 11.

Avantageusement, la section transversale de chaque canal courbe 14, 15, 16 se réduit progressivement entre le canal central 12 et la chambre tourbillonnaire 19. Ceci permet notamment d'accélérer les flux s'écoulant dans lesdits canaux courbes, optimisant leur vitesse lorsqu'ils débouchent dans la chambre tourbillonnaire 19.

Avantageusement, les au moins deux canaux courbes 14, 15, 16 sont de formes, dimensions et courbures sensiblement identiques entre le canal central 12 et la chambre tourbillonnaire 19. Néanmoins, en variante, on pourrait envisager de réaliser des canaux courbes de formes, dimensions, notamment longueur et section, et courbures différentes, générant ainsi des débits de flux différents dans chaque canal courbe. Associée à une répartition non régulière des canaux courbes autour de la chambre tourbillonnaire, ceci pourrait permettre de réaliser des sprays d'une forme quelconque souhaitée.

Comme évoqué précédemment, l'invention pourrait aussi s'appliquer à un dispositif du type bidose. Dans ce cas, le contenu du réservoir serait distribué en deux actionnements successifs. Le document WO2014147329 décrit un exemple de dispositif bidose. Plus généralement, la présente invention s'applique à tous types de dispositifs unidose, bidose ou multidose.

Selon un aspect avantageux de l'invention, la tête de pulvérisation 10 est fabriquée par impression additive ou impression 3D. Ce procédé de fabrication permet de réaliser des formes monoblocs complexes, tels que la tête de pulvérisation 10 incluant le profil de pulvérisation 13 avec les canaux courbes 14, 15, 16, en une seule pièce monobloc, sans assemblage d'un ou plusieurs insert(s), et avec des performances parfaitement reproductibles d'une tête de pulvérisation à l'autre.

La présente invention fournit plusieurs avantages, notamment :
- elle améliore la reproductibilité du spray, chaque tête de pulvérisation comportant un profil de pulvérisation identique;
- elle évite d'avoir à assembler deux ou plusieurs pièces fabriquées séparément, supprimant une étape de fabrication et une étape d'assemblage;
- elle permet de réaliser des formes complexes quelconques souhaitées, par l'utilisation de l'impression additive;
- elle permet d'optimiser les propriétés du spray grâce à une géométrie optimisée du profil de pulvérisation.

Bien entendu, d'autres variantes de réalisation sont également envisageables, sans sortir du cadre de la présente invention, tel que défini par les revendications annexées.

## Revendications

1. Tête de pulvérisation (10) pour dispositif de distribution de produit fluide, comportant un corps (18) pourvu d'un orifice de pulvérisation (11), un canal central (12) étant relié audit orifice de pulvérisation (11) via un profil de pulvérisation (13) comportant une chambre tourbillonnaire (19) disposée directement en amont dudit orifice de pulvérisation (11), **caractérisée en ce que** ledit profil de pulvérisation (13) comporte au moins deux canaux courbes (14, 15, 16) s'étendant à partir dudit canal central (12) vers ladite chambre tourbillonnaire (19), chaque canal courbe (14, 15, 16) se connectant de manière non radiale à ladite chambre tourbillonnaire (19), pour former un spray en sortie dudit orifice de pulvérisation (11), chaque canal courbe (14, 15, 16) étant de forme convexe, en s'écartant latéralement vers l'extérieur à partir dudit canal central (12), puis à partir d'une zone d'inflexion (140, 150, 160), en revenant latéralement vers l'intérieur en direction de ladite chambre tourbillonnaire (19).

2. Tête de pulvérisation (10) selon la revendication 1, dans laquelle ladite tête de pulvérisation est du type nasal, ledit canal central (12) s'étendant axialement et ledit orifice de pulvérisation (11) étant orienté axialement.

3. Tête de pulvérisation (10) selon la revendication 1 ou 2, dans laquelle lesdits au moins deux canaux courbes (14, 15, 16) débouchent dans ladite chambre tourbillonnaire (19) en étant régulièrement répartis sur la périphérie de ladite chambre tourbillonnaire (19).

4. Tête de pulvérisation (10) selon l'une quelconque des revendications précédentes, dans laquelle chaque canal courbe (14, 15, 16) est de forme arrondie, sans aucun angle vif.

5. Tête de pulvérisation (10) selon l'une quelconque des revendications précédentes, dans laquelle la section transversale de chaque canal courbe (14, 15, 16) se réduit progressivement entre ledit canal central (12) et ladite chambre tourbillonnaire (19).

6. Tête de pulvérisation (10) selon l'une quelconque des revendications précédentes, dans laquelle lesdits au moins deux canaux courbes (14, 15, 16) sont de formes, dimensions et courbures sensiblement identiques entre ledit canal central (12) et ladite chambre tourbillonnaire (19).

7. Tête de pulvérisation (10) selon l'une quelconque des revendications 1 à 5, dans laquelle lesdits au moins deux canaux courbes (14, 15, 16) sont de formes, dimensions et/ou courbures différentes entre ledit canal central (12) et ladite chambre tourbillonnaire (19).

8. Tête de pulvérisation (10) selon l'une quelconque des revendications précédentes, dans laquelle ledit profil de pulvérisation (13) comporte deux canaux courbes (14, 15).

9. Tête de pulvérisation (10) selon l'une quelconque des revendications 1 à 7, dans laquelle ledit profil de pulvérisation (13) comporte trois canaux courbes (14, 15, 16).

10. Dispositif de distribution de produit fluide, comportant une partie d'actionnement (1), un réservoir (20) contenant un produit fluide, une tête de pulvérisation (10) pourvue d'un orifice de pulvérisation (11), et des moyens de distribution (30) pour distribuer au moins une partie dudit produit fluide à travers ledit orifice de pulvérisation (11) lors d'un actionnement, **caractérisé en ce que** ladite tête de pulvérisation (10) est réalisée selon l'une quelconque des revendications précédentes.

11. Dispositif selon la revendication 10, dans lequel ledit réservoir (20) contient une seule dose de produit fluide distribuée en un seul actionnement du dispositif.

12. Dispositif selon la revendication 10, dans lequel ledit réservoir (20) contient au moins deux doses de produit fluide distribuées en plusieurs actionnements successifs du dispositif.

13. Dispositif selon l'une quelconque des revendications 10 à 12, dans lequel ladite partie d'actionnement (1) comporte deux éléments d'appui latéraux (2, 3) montés pivotants sur ladite tête de pulvérisation (10), chaque élément d'appui latéral (2, 3) comportant une partie de came respective (21, 31) comportant chacune une surface de came respective (22, 32) coopérant avec ledit réservoir (20) pour le déplacer axialement vers le haut lors de l'actionnement du dispositif.

14. Procédé de fabrication d'une tête de pulvérisation (10) selon l'une quelconque des revendications 1 à 9, **caractérisé en ce que** ledit procédé comprend de réaliser ladite tête de pulvérisation (10) par impression additive.

## Patentansprüche

1. Sprühkopf (10) für eine Vorrichtung zur Abgabe eines Fluidprodukts, enthaltend einen Körper (18) mit einer Sprühöffnung (11), wobei ein zentraler Kanal (12) mit der Sprühöffnung (11 ) über ein Sprühprofil (13) verbunden ist, das eine Wirbelkammer (19) umfasst, die der Sprühöffnung (11) direkt vorgelagert ist,
**dadurch gekennzeichnet, dass** das Sprühprofil (13) zumindest zwei gekrümmte Kanäle (14, 15, 16) aufweist, die sich von dem zentralen Kanal (12) zur Wirbelkammer (19) erstrecken, wobei jeder gekrümmte Kanal (14, 15, 16) nicht-radial mit der Wirbelkammer (19) verbunden ist, um am Ausgang der Sprühöffnung (11) ein Spray zu bilden, wobei jeder gekrümmte Kanal (14, 15, 16) konvex geformt ist und dabei von dem zentralen Kanal (12) seitlich nach außen führt und dann von einem Biegungsbereich (140, 150, 160) aus seitlich nach innen in Richtung der Wirbelkammer (19) zurückkehrt.

2. Sprühkopf (10) nach Anspruch 1,
wobei der Sprühkopf zur nasalen Anwendung ausgelegt ist, wobei sich der zentrale Kanal (12) axial erstreckt und die Sprühöffnung (11) axial ausgerichtet ist.

3. Sprühkopf (10) nach Anspruch 1 oder 2,
wobei die zumindest zwei gekrümmten Kanäle (14, 15, 16) in die Wirbelkammer (19) münden und dabei gleichmäßig über den Umfang der Wirbelkammer (19) verteilt sind.

4. Sprühkopf (10) nach einem der vorhergehenden Ansprüche,
wobei jeder gekrümmte Kanal (14, 15, 16) eine abgerundete Form ohne scharfe Ecken aufweist.

5. Sprühkopf (10) nach einem der vorhergehenden Ansprüche,
wobei sich der Querschnitt jedes gekrümmten Kanals (14, 15, 16) zwischen dem zentralen Kanal (12) und der Wirbelkammer (19) allmählich verjüngt.

6. Sprühkopf (10) nach einem der vorhergehenden Ansprüche,
wobei die zumindest zwei gekrümmten Kanäle (14, 15, 16) zwischen dem Zentralkanal (12) und der Wirbelkammer (19) im Wesentlichen die gleiche Form, Abmessung und Krümmung aufweisen.

7. Sprühkopf (10) nach einem der Ansprüche 1 bis 5,
wobei die zumindest zwei gekrümmten Kanäle (14, 15, 16) zwischen dem zentralen Kanal (12) und der Wirbelkammer (19) unterschiedliche Formen, Abmessungen und/oder Krümmungen aufweisen.

8. Sprühkopf (10) nach einem der vorhergehenden Ansprüche,
wobei das Sprühprofil (13) zwei gekrümmte Kanäle (14, 15) aufweist.

9. Sprühkopf (10) nach einem der Ansprüche 1 bis 7,
wobei das Sprühprofil (13) drei gekrümmte Kanäle (14, 15, 16) aufweist.

10. Vorrichtung zur Abgabe eines Fluidprodukts, umfassend ein Betätigungsteil (1), ein Reservoir (20), das ein Fluidprodukt enthält, einen Sprühkopf (10) mit einer Sprühöffnung (11), und Abgabemittel (30), um bei einer Betätigung zumindest einen Teil des Fluidprodukts durch die Sprühöffnung (11) abzugeben,
**dadurch gekennzeichnet, dass** der Sprühkopf (10) nach einem der vorhergehenden Ansprüche ausgeführt ist.

11. Vorrichtung nach Anspruch 10,
wobei das Reservoir (20) eine einzelne Dosis an Fluidprodukt enthält, die bei einer einzelnen Betätigung der Vorrichtung abgegeben wird.

12. Vorrichtung nach Anspruch 10,
wobei das Reservoir (20) zumindest zwei Dosen an Fluidprodukt enthält, die in mehreren aufeinanderfolgenden Betätigungen der Vorrichtung abgegeben werden.

13. Vorrichtung nach einem der Ansprüche 10 bis 12,
wobei das Betätigungsteil (1) zwei seitliche Auflageelemente (2, 3) aufweist, die schwenkbar an dem Sprühkopf (10) gelagert sind, wobei jedes seitliche Auflageelement (2, 3) einen jeweiligen Nockenabschnitt (21, 31) mit jeweils einer entsprechenden Nockenfläche (22, 32) aufweist, die mit dem Reservoir (20) zusammenwirkt, um dieses bei der Betätigung der Vorrichtung axial nach oben zu bewegen.

14. Verfahren zur Herstellung eines Sprühkopfes (10) nach einem der Ansprüche 1 bis 9,
**dadurch gekennzeichnet, dass** das Verfahren das Herstellen des Sprühkopfes (10) durch additives Drucken umfasst.

## Claims

1. spray head (10) for a device for dispensing a fluid product, comprising a body (18) provided with a spray orifice (11), a central channel (12) being connected to said spray orifice (11) via a spray profile (13) comprising a vortex chamber (19) arranged directly upstream of said spray orifice (11), **characterised in that** said spray profile (13) comprises at least two curved channels (14, 15, 16) extending from said central channel (12) to said vortex chamber (19), each curved channel (14, 15, 16) connected non-radially to said vortex chamber (19), to form a spray exiting said spray orifice (11), each curved channel (14, 15, 16) having a convex shape, diverging laterally outwards from said central channel (12), and then from an inflection point (140, 150, 160), returning laterally inwards towards said vortex chamber (19).

2. The spray head (10) according to according claim 1, wherein said spray head is of the nasal type, said central channel (12) extending axially and said spray orifice (11) being orientated axially.

3. The spray head (10) according to claim 1 or 2, wherein said at least two curved channels (14, 15, 16) open out into said vortex chamber (19) while being regularly distributed over the periphery of said vortex chamber (19).

4. The spray head (10) according to any one of the preceding claims, wherein each curved channel (14, 15, 16) is rounded in shape without any sharp corners.

5. The spray head (10) according to any one of the preceding claims, wherein the cross-section of each curved channel (14, 15, 16) is reduced progressively between said central channel (12) and said vortex chamber (19).

6. The spray head (10) according to any one of the preceding claims, wherein said at least two curved channels (14, 15, 16) are substantially identical in shape, dimension and curvature between said central channel (12) and said vortex chamber (19).

7. The spray head (10) according to any one of claims 1 to 5, wherein the said at least two curved channels (14, 15, 16) are different in shape, dimension and/or curvature between said central channel (12) and said vortex chamber (19).

8. The spray head (10) according to any one of the preceding claims, wherein said spray profile (13) comprises two curved channels (14, 15).

9. The spray head (10) according to any one of claims 1 to 7, wherein said spray profile (13) comprises three curved channels (14, 15).

10. The device for dispensing a fluid product, comprising an actuation portion (1); a reservoir (20) containing a fluid product; a spray head provided with a spray orifice (11); and a dispenser means (30) for dispensing at least a portion of said fluid product through said spray orifice (11) during actuation, **characterised in that** said spray head (10) is manufactured according to any one of the preceding claims.

11. The device according to claim 10, wherein said reservoir (20) contains a single dose of fluid product dispensed in a single actuation of the device.

12. The device according to claim 10, wherein said reservoir (20) contains at least two doses of fluid product dispensed during a plurality of successive actuations of the device.

13. The device according to any one of claims 10 to 12, wherein said actuation portion (1) comprises two lateral presser elements (2, 3) mounted to pivot on said spray head (10), each lateral presser element (2, 3) comprising a respective cam portion (21, 31), each comprising a respective cam surface (22, 32) co-operating with said reservoir (20) to move it axially upwards while the device is being actuated.

14. The method of manufacturing a spray head (10) according to any one of Claims 1 to 9, **characterised in that** said method comprises making said spray head (10) by additive printing.
